# EUROPÄISCHE PATENTSCHRIFT

(11) **EP 0 710 093 B1**
(45) Veröffentlichungstag und Bekanntmachung des Hinweises auf die Patenterteilung: **02.09.1998**
(21) Anmeldenummer: 94919459.1
(22) Anmeldetag: 30.06.1994
(51) Int. Cl.: A61F 5/01

(54) **SCHWENKBAR MITEINANDER VERBUNDENE KNIEFÜHRUNGSSCHIENEN**
KNEE SPLINTS SWIVELLINGLY LINKED TO EACH OTHER
ECLISSES POUR GENOU RELIEES PIVOTANTES

(30) Priorität: 20.07.1993 AT 1440/93; 21.10.1993 AT 2129/93
(43) Veröffentlichungstag der Anmeldung: 08.05.1996
(73) Patentinhaber: GRAFINGER, Josef, A-1160 Wien (AT)
(72) Erfinder: GRAFINGER, Josef, A-1160 Wien (AT)
(74) Vertreter: Rippel, Andreas, Dipl.-Ing.
(86) Internationale Anmeldenummer: AT9400084
(87) Internationale Veröffentlichungsnummer: WO9503013

(56) Entgegenhaltungen:
- FR-A- 2 600 528
- GB-A- 2 235 012
- US-A- 3 779 654
- US-A- 4 463 751
- US-A- 5 009 223
- US-A- 5 107 824

## Beschreibung

Die Erfindung bezieht sich auf schwenkbar miteinander verbundene Knieführungsschienen zur Befestigung an Schalen oder Bändern von Kniegelenkorthesen, Stützapparaten oder an Unterschenkelprothesen, wobei in einer ersten Schiene zwei bogenförmige Führungen vorgesehen sind, in die den Schwenkwinkel der beiden Schienen begrenzende Bolzen der zweiten Schiene eingreifen.

In der frühfunktionellen Behandlung des Kniegelenkes werden Orthesen mit begrenzbarer Gelenkbewegung eingesetzt. Der Einsatz erfolgt dabei z.B. postoperativ in der Kniegelenkschirurgie; zur Immobilisierung nach Knieverletzungen zweiten Grades die keine Operation verlangen und zur späteren Mobilisierung; postoperativ anstelle eines Gipses zur Vermeidung einer Subluxation der Tibia nach anteriorer und posteriorer Kreuzbandverletzung- oder Rekonstruktion; zur sicheren Stabilisierung der Verletzten oder operativ versorgten Seitenbänder.

In vielen Fällen wird der Bewegungsspielraum , sowohl der Extension als auch der Flexion, durch Versetzen von Schrauben auf einer Anschlagplatte begrenzt. Es sind aber auch Orthesen mit freier Gelenkbewegung bekannt.

Um eine größtmögliche Anpassung der gegenseitigen Bewegung der Knieführungsschienen an die natürliche Bewegung des Kniegelenkes zu erreichen, wurden schon die verschiedensten Anordnungen vorgeschlagen und auch verwirklicht. Insbesondere sind Doppelgelenk-Knieführungsschienen in Gebrauch, die als Doppelgelenk-Zahnsegmentschienen ausgeführt sind. Dabei sind beide Schienen mit einem Zahnsegment versehen, welche Segmente miteinander kämmen, und zur Verbindung ist eine gelenkig mit beiden Schienen verbundene Zwischenplatte angeordnet.

Wie jedoch eingehende Untersuchungen gezeigt haben, können auch diese Orthesen den natürlichen Bewegungsablauf des Kniegelenkes nicht nachvollziehen.

Aus der AT-PS 393 620 ist eine Knieführungsschiene bekannt geworden, bei der in einer ersten Schiene zwei bogenförmige Führungen vorgesehen sind, in die den Schwenkwinkel der beiden Schienen begrenzende Zapfen der zweiten Schiene eingreifen. Die Zapfen oder Bolzen stellen dabei in der jeweiligen Endstellung die Achse für die kreisbogenförmige Führung des anderen Zapfens dar.

Eine derartige Anordnung vermag nicht die natürliche Bewegung des Knies nachzuahmen, die durch eine kombinierte Dreh- und Gleitbewegung gebildet wird.

Aus der US-A-5 009 223 sind Knieführungsschienen der eingangs genannten Art bekannt, bei denen zusätzlich zu den beiden bogenförmigen Führungen zwei weitere bogenförmige Führungen angeordnet sind. Diese beiden zusätzlichen Führungen bilden eine Art 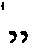 T", wobei in einem Steg des 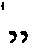 T" ein Bolzen, im anderen Steg des 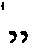 T" ein weiterer Bolzen der anderen Schiene geführt ist. Auch bei dieser bekannten Anordnung ist eine dem natürlichen Bewegungsablauf entsprechende Führung nicht möglich.

Die Erfindung hat es sich daher zum Ziel gesetzt, schwenkbar miteinander verbundene Knieführungsschienen zu schaffen, die den natürlichen Bewegungsablauf des Kniegelenkes genauer nachvollziehen können als die bisher bekannten Knieführungsschienen. Erreicht wird dies, ausgehend von einer Knieführungsschiene der eingangs genannten Art, dadurch, daß ausschließlich eine zusätzliche Führung vorgesehen ist, die dadurch gebildet wird daß auschließlich ein in der ersten Schiene in einer Bohrung sitzender Bolzen in diese zusätzliche bogenförmige Führung der zweiten Schiene eingreift, die sich über die Längsachse der zweiten Schiene (2) und schräg zu ihr erstreckt.

Bei einer erfindungsgemäßen Knieführungsschiene sind demnach drei bogenförmige Führungen vorgesehen, die zwangsläufig nicht kreisbogenförmig ausgebildet sind. Dadurch wird auf einfache Weise eine optimale Annäherung an die natürliche Bewegung des Knies erreicht.

Schwenkbar miteinander verbundene Knieführungsschienen mit den erfindungsgemässen Merkmalen können auch für Unterschenkelprothesen verwendet werden, wenn der Unterschenkelstumpf so kurz ist, daß auch eine Festlegung der Prothese am Oberschenkel notwendig ist.

Bei Orthesen, bei denen eine Begrenzung der Extension und/oder Flexion erforderlich ist, ist nach einem ausgestaltenden Merkmal der Erfindung zur Begrenzung der Extension und/oder Flexion die zweite Schiene mit einer Anzahl von Bohrungen zum wahlweisen Einsetzen der in die zwei weiteren bogenförmigen Führungen eingreifenden, als Anschläge wirkenden Bolzen versehen.

Eine bevorzugte Ausführungsform der Erfindung zeichnet sich dadurch aus, daß in der gestreckten Stellung der Knieführungsschienen die Enden der bogenförmigen Führung der zweiten Schiene zum inneren Mittelbereich der bogenförmigen Führungen der ersten Schiene weisen.

Zahlreiche Versuche und Untersuchungen haben gezeigt, daß der natürliche Bewegungsablauf des Kniegelenkes noch besser nachvollzogen werden kann, wenn die sich schräg zur Längsachse der zweiten Schiene erstreckende bogenförmige Führung eine Krümmung aufweist, deren konvexe Seite in abgewinkelter Stellung der Knieführungsschienen in Richtung zur ersten Schiene weist.

Eine bevorzugte Ausführungsform der Erfindung zeichnet sich dadurch aus, daß die bogenförmige Führung sich in Richtung von der zweiten Schiene weg leicht erweitert.

Nachstehend ist die Erfindung anhand von in den Zeichnungen dargestellten Ausführungsbeispielen näher beschrieben, ohne auf diese Beispiele beschränkt zu sein. Dabei zeigen: Fig. 1 eine Oberschiene, d.h. eine am Oberschenkel in bekannter Weise mit Schalen oder Bändern zu befestigende Schiene einer ersten Ausführungsform; Fig. 2 die zugehörige Unterschiene; Fig. 3 die beiden miteinander verbundenen Schienen in einer Zwischenstellung; Fig. 4 die beiden miteinander verbundenen Schienen in gestreckter Stellung; Fig. 5 eine Oberschiene einer zweiten Ausführungsform; Fig. 6 die zugehörige Unterschiene, wobei die Umrisse der Oberschiene strichliert angedeutet sind und Fig. 7 die bogenförmige Führung allein, wobei Maße eingetragen sind.

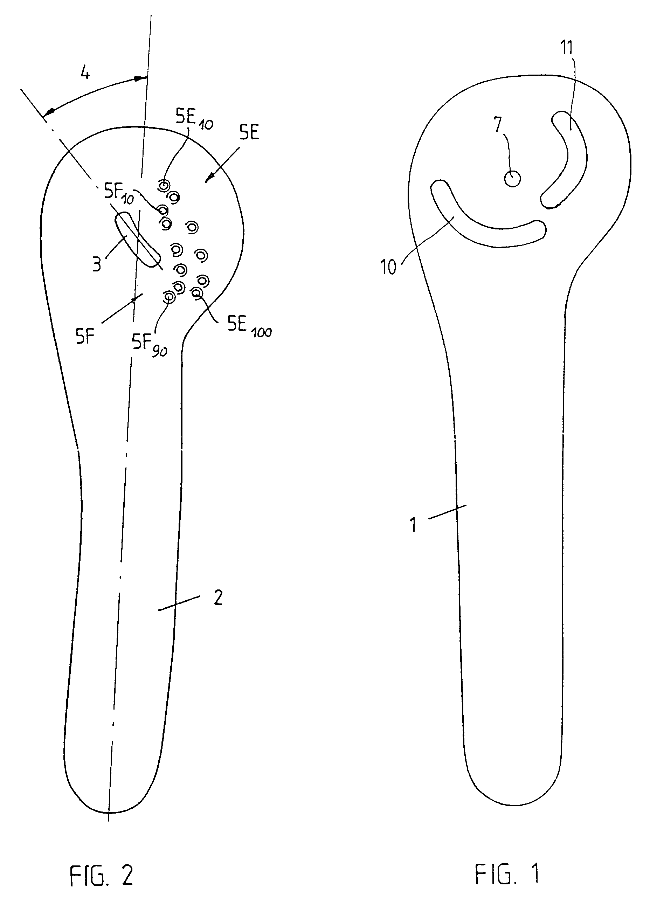

Gemäß den Fig. 1 bis 4 ist eine Oberschiene 1 und eine Unterschiene 2 vorgesehen. Die Unterschiene 2 weist eine bogenförmige Führung 3 auf, die schräg zur Längsachse der Unterschiene 2 und damit zur Längsachse des Unterschenkels verläuft. Der Winkel 4 zwischen Längsachse der Unterschiene 2 und Führung 3 beträgt im Beispiel etwa 40°.

Eine Reihe von Gewindebohrungen 5 in der Unterschiene 2 ermöglicht das Einsetzen von Bolzen 6. Die Bohrungen 5E dienen, soferne Bolzen 6 eingesetzt sind, der Begrenzung der Extension, die Bohrungen 5F, soferne Bolzen 6 eingesetzt sind, der Begrenzung der Flexion.

Die Oberschiene 1 ist mit einer Bohrung 7 versehen, in die eine von einem Bolzen 8 gehaltene Büchse 9 eingesetzt ist, die in der bogenförmigen Führung 3 gleiten kann.

Die Köpfe der Bolzen 6 gleiten in bogenförmigen Führungen 10 und 11 an der Oberschiene 1 und führen im Zusammenwirken mit der Führung 3 bzw. der Büchse 9 die beiden Schienen 1 und 2 gegeneinander derart, daß die natürliche Bewegung im Knie nachvollzogen wird.

Gleichzeitig begrenzen die Bolzen 6 bzw. deren Köpfe die Bewegung der beiden Schienen 1 und 2 gegeneinander.

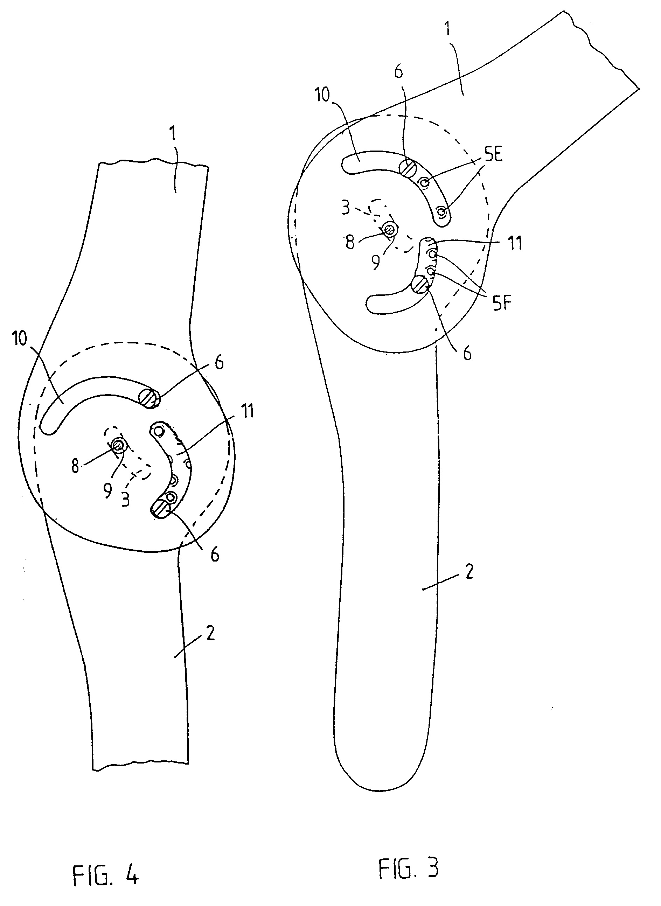

In Fig. 2 sind aus Gründen der Übersichtlichkeit nur einzelne der Gewindebohrungen 5E, 5F der genauen Gradeinteilung zugeordnet. So begrenzt ein in die Gewindebohrung 5E₁₀ eingesetzter Bolzen 6 die Extension auf 10°, ein in die Gewindebohrung 5E₁₀₀ eingesetzter Bolzen die Extension auf 100° und ein in die Gewindebohrung 5F₁₀ eingesetzter Bolzen die Flexion auf 10°. Die in den Fig. 3 und 4 eingezeichnete Lage der Bolzen 6 würde daher einer Extension von 10° und einer Flexion von 90° entsprechen.

Wie aus Fig. 4 ersichtlich ist, weisen in der dort gezeigten gestreckten Stellung der Knieführungsschienen 1,2 die Enden der bogenförmigen Führung 3 der zweiten Schiene 2 zum inneren Mittelbereich der bogenförmigen Führungen 10,11 der ersten Schiene 1.

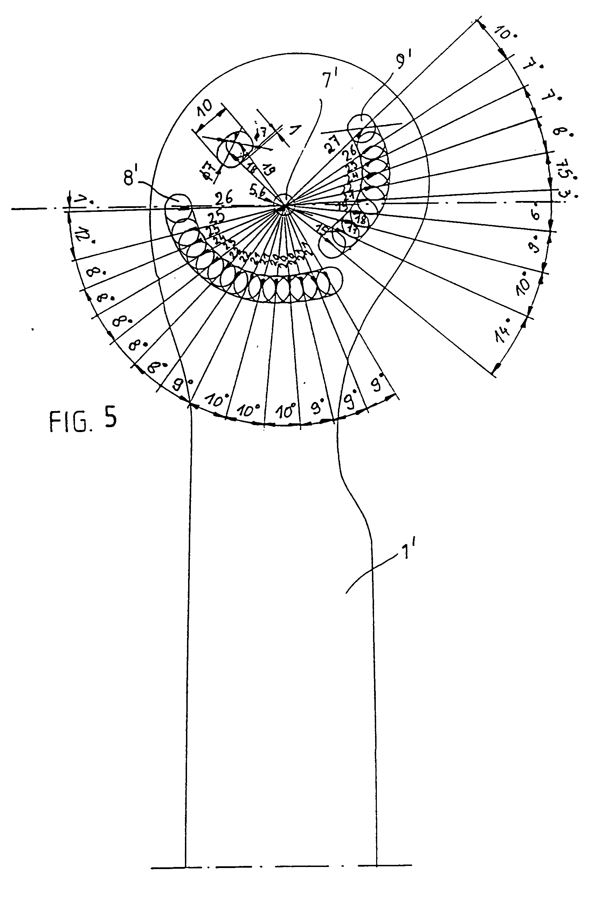

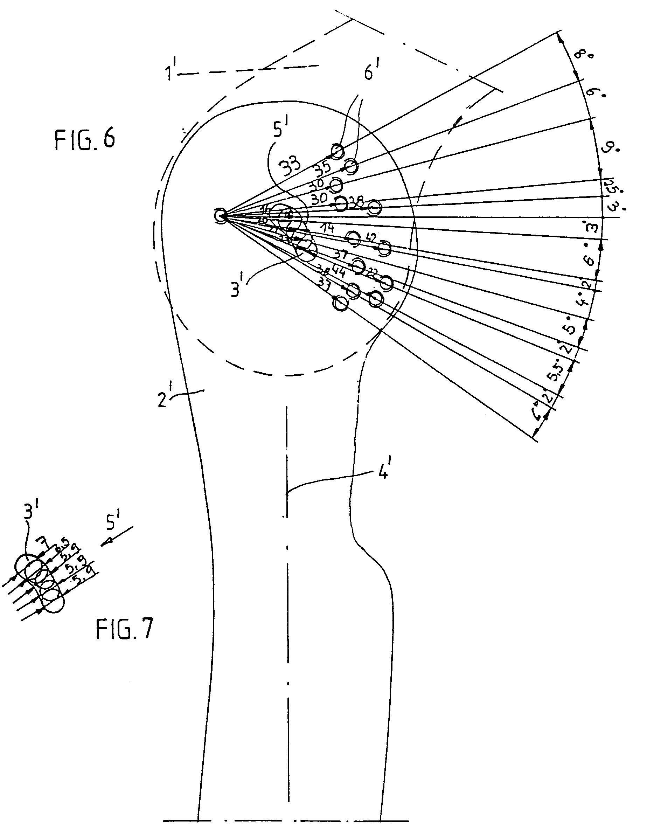

Gemäß den Figuren 5 und 6 ist eine Oberschiene 1' und eine Unterschiene 2' vorgesehen. Die Unterschiene 2' weist eine bogenförmige Führung 3' auf, die schräg zur Längsachse 4' der Unterschiene 2' und damit zur Längsachse des Unterschenkels verläuft.

Wie aus Fig. 6 ersichtlich ist, weist die konvexe Seite 5' der bogenförmigen Führung 3' in Richtung zur Oberschiene 1'.

Aus den Maßen nach Fig. 7 ergibt sich, daß die bogenförmige Führung 3' sich in Richtung von der Unterschiene weg leicht erweitert. Die Maße hängen natürlich vom Durchmesser des in die Führung 3' eingreifenden Bolzens ab, der aus Gründen der Übersichtlichkeit nicht dargestellt ist.

Wie beim vorigen Ausführungsbeispiel ermöglicht eine Reihe von Gewindebohrungen 6' in der Unterschiene 2' das Einsetzen von Bolzen, wobei die Bohrungen 6', soferne Bolzen eingesetzt sind, der Begrenzung der Extension und der Begrenzung der Flexion dienen.

Die Oberschiene 1' ist mit einer Bohrung 7' versehen, in die eine von einem Bolzen gehaltene Büchse eingesetzt ist, die in der bogenförmigen Führung 3' gleiten kann. Weiters sind in der Oberschiene 1' Führungen 8' und 9' angeordnet, in die die in die Bohrungen 6' eingesetzten Bolzen bzw. deren Bolzenköpfe eingreifen. Im Zusammenwirken mit der Führung 3' werden die Schienen 1',2' dadurch so geführt, daß die natürliche Bewegung im Knie perfekt nachvollzogen wird.

In den Zeichnungen sind auch die Maße eines bevorzugten Ausführungsbeispieles eingetragen, auf das die Erfindung jedoch nicht beschränkt ist.

Im Rahmen der Erfindung sind zahlreiche Abänderungen gegenüber den gezeigten Ausführungsbeispielen möglich. So könnte, um eine leichte Bewegung zu erreichen, statt des Gewindebolzens und der Buchse ein Nadellager verwendet werden, dessen Außenring in der etwa bogenförmigen Führung 3 bzw. 3' verschiebbar ist.

## Patentansprüche

1. Schwenkbar miteinander verbundene Knieführungsschienen zur Befestigung an Schalen oder Bändern von Kniegelenkorthesen, Stützapparaten oder an Unterschenkelprothesen, wobei in einer ersten Schiene (1) zwei bogenförmige Führungen (10,11) vorgesehen sind, in die den Schwenkwinkel der beiden Schienen begrenzende Bolzen (6) der zweiten Schiene (2) eingreifen, **dadurch gekennzeichnet**, daß ausschließlich eine zusätzliche Führung vorgesehen ist, die dadurch gebildet wird, daß auschließlich ein in der ersten Schiene (1) in einer Bohrung (7) sitzender Bolzen (8) in diese zusätzliche bogenförmige Führung (3) der zweiten Schiene (2) eingreift, die sich über die Längsachse der zweiten Schiene (2) und Schräg zu ihr erstreckt.

2. Schwenkbar miteinander verbundene Knieführungsschienen nach Anspruch 1, **dadurch gekennzeichnet**, daß zur Begrenzung der Extension und/oder Flexion die zweite Schiene (2) mit einer Anzahl von Bohrungen (5) zum wahlweisen Einsetzen der in die zwei bogenförmigen Führungen (10,11) eingreifenden, als Anschläge wirkenden Bolzen (6) versehen ist.

3. Schwenkbar miteinander verbundene Knieführungsschienen nach Anspruch 1 oder 2, **dadurch gekennzeichnet**, daß in der gestreckten Stellung der Knieführungsschienen (1,2) die Enden der zusätzlichen bogenförmigen Führung (3) zum inneren Mittelbereich der bogenförmigen Führungen (10,11) der ersten Schiene (1) weisen.

4. Schwenkbar miteinander verbundene Knieführungsschienen nach einem der Ansprüche 1 bis 3, **dadurch gekennzeichnet**, daß die sich schräg zur Längsachse der zweiten Schiene (2') erstreckende bogenförmige Führung (3') eine Krümmung aufweist, deren konvexe Seite (5') in abgewinkelter Stellung der Knieführungsschienen (1',2') in Richtung zur ersten Schiene (1') weist.

5. Schwenkbar miteinander verbundene Knieführungsschienen nach Anspruch 1, **dadurch gekennzeichnet**, daß die bogenförmige Führung (3') sich in Richtung von der zweiten Schiene (2') weg leicht erweitert.

## Claims

1. Knee guide bars, pivotally connected together, on cups or belts of prosthetic knee joints, support devices or on lower-leg prostheses, there being provided in a first bar (1), two arcuate guides (10,11) in which bolts (6) of the second bar (2), limiting the pivoting angle of both bars, engage, characterised in that only one additional guide is provided which is formed in that only one bolt (8) sitting in a bore (7) in the first bar (1) engages in this additional arcuate guide (3) of the second bar (2), which extends over the longitudinal axis of the second bar (2) and obliquely thereto.

2. Knee guide bars, pivotally connected together, according to claim 1, characterised in that, in order to limit the extension and/or flexion, the second bar (2) is provided with a number of bores (5) for optional insertion of the bolts (6), acting as stops and engaging in the two arcuate guides (10,11).

3. Knee guide bars, pivotally connected together, according to claim 1 or 2, characterised in that, in the extended position of the knee guide bars (1, 2) the ends of the additional arcuate guide (3) are aligned towards the inner central area of the arcuate guides (10,11) of the first bar (1) .

4. Knee guide bars, pivotally connected together, according to one of claims 1 to 3, characterised in that the arcuate guide (3') extending obliquely to the longitudinal axis of the second bar (2') has a curvature the convex side (5') of which, in the angled position of the knee guide bars (1', 2'), is aligned in the direction of the first bar (1') .

5. Knee guide bars, pivotally connected together, according to claim 1, characterised in that the arcuate guide (3') expands slightly in the direction leading away from the second bar (2').

## Revendications

1. Rails de guidage du genou raccordés entre eux de façon à pouvoir pivoter et destinés à être fixés à des coquilles ou bandes de supports orthopédiques de l'articulation du genou, d'appareils de soutien, ou à des prothèses de la jambe, dans lesquels on a prévu, dans un premier rail (1), deux glissières de guidage (10, 11) en forme d'arcs dans lesquelles s'engagent des boulons (6) du second rail (2) qui limitent l'angle de rotation des deux rails, caractérisés en ce qu'on prévoit exclusivement une glissière de guidage supplémentaire qui est formée exclusivement par le fait qu'un boulon (8), en appui dans le premier rail (1), dans un perçage (7), s'engage dans cette glissière de guidage (3) supplémentaire en forme d'arc du second rail (2) qui s'étend sur l'axe longitudinal du second rail (2) et en oblique par rapport à celui-ci.

2. Rails de guidage du genou raccordés entre eux de façon à pouvoir pivoter, selon la revendication 1, caractérisés en ce que pour limiter l'extension et/ou la flexion, le second rail (2) est pourvu d'un certain nombre de perçages (5) permettant l'introduction facultative des boulons (6) qui s'engagent dans les deux glissières de glissière de guidage (10, 11) en forme d'arcs et font fonction de butées.

3. Rails de guidage du genou raccordés entre eux de façon à pouvoir pivoter, selon la revendication 1 ou 2, caractérisés en ce que dans la position étirée des rails de guidage du genou (1, 2), les extrémités de la glissière de guidage supplémentaire (3) en forme d'arc sont pointées vers la zone médiane intérieure des glissières de guidage en forme d'arcs (10, 11) du premier rail (1) .

4. Rails de guidage du genou raccordés entre eux de façon a pouvoir pivoter, selon l'une des revendications 1 à 3, caractérisés en ce que la glissière de guidage en forme d'arc (3') s'étendant en oblique par rapport à l'axe longitudinal du second rail (2') présente une courbure dont le côté convexe (5') est tourné dans la direction du premier rail (1') lorsque les rails de guidage du genou (1', 2') sont en position inclinée.

5. Rails de guidage du genou raccordés entre eux de façon à pouvoir pivoter, selon la revendication 1, caractérisés en ce que la glissière de guidage en forme d'arc (3') s'élargit légèrement dans la direction de son écartement du second rail (2') .
